(19) Europäisches Patentamt
European Patent Office
Office européen des brevets

(11) Publication number: **0 223 593 B1**

# (12) EUROPEAN PATENT SPECIFICATION

(45) Date of publication of patent specification: **07.08.91**

(51) Int. Cl.⁵: **C07C 311/00**, C07D 333/34, A61K 31/18, A61K 31/63

(21) Application number: **86308992.6**

(22) Date of filing: **18.11.86**

## (54) Phenoxyalkylcarboxylic acids and esters and their preparation and pharmaceutical formulation.

(30) Priority: **19.11.85 GB 8528398**

(43) Date of publication of application:
**27.05.87 Bulletin 87/22**

(45) Publication of the grant of the patent:
**07.08.91 Bulletin 91/32**

(84) Designated Contracting States:
**AT BE CH DE ES FR GB GR IT LI LU NL SE**

(56) References cited:
**EP-A- 0 004 011**
**EP-A- 0 239 907**

(73) Proprietor: **GLAXO GROUP LIMITED**
**Clarges House 6-12 Clarges Street**
**London W1Y 8DH(GB)**

(72) Inventor: **Hallet, Peter**
**46 Walnut Tree Close Bassingbourn**
**Royston Hertfordshire(GB)**

(74) Representative: **Skailes, Humphrey John et al**
**Frank B. Dehn & Co. Imperial House 15-19 Kingsway**
**London WC2B 6UZ(GB)**

EP 0 223 593 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to phenoxyalkylcarboxylic acid and ester compounds, to processes for their preparation, to pharmaceutical compositions containing them and to their use in medicine.

The compounds of the invention have good thromboxane $A_2$ as well as prostaglandin $G_2$ and $H_2$ antagonist activity and in particular inhibit blood platelet aggregation, bronchoconstriction and vasoconstriction. The new compounds are thus of special interest in the treatment of asthma and occlusive vascular diseases.

Thus the present invention provides compounds of the general formula (1)

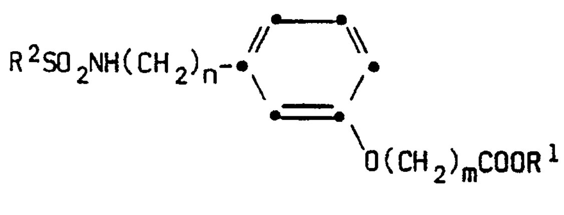

(1)

wherein

$R^1$ is a hydrogen atom or a $C_{1-4}$ alkyl group;

$R^2$ is a naphthyl, thienyl or $\beta$-styryl group or a group $Ar(CH_2)_p$- where p is zero or an integer from 1 to 3 and Ar is phenyl or phenyl substituted by one or more halogen atoms or $C_{1-4}$ alkyl, $C_{1-4}$ alkoxy, hydroxyl, trifluoromethyl, nitro or amino groups; m is an integer 1 or 2;

n is an integer from 1 to 4;

and the physiologically acceptable salts thereof.

Similar compounds in which the two substituents on the benzene ring are in the para-position, are described in EP-A-4011.

Suitable physiologically acceptable salts of the compounds of general formula (1) include salts formed with bases when $R^1$ is a hydrogen atom. Examples of such salts are alkali metal (e.g. sodium or potassium), alkaline earth metal (e.g. calcium or magnesium), ammonium and substituted ammonium (e.g. triethylammonium and piperidine). When $R^2$ is phenyl substituted by amino, salts may also be formed with inorganic or organic acids, e.g. hydrochlorides.

In general formula (1) the group $R^1$ may be for example a methyl or ethyl group. $R^1$ is preferably however a hydrogen atom or a methyl group, particularly a hydrogen atom.

The chain $-(CH_2)_n-$ in compounds of formula (1) may be $-CH_2-$, $-(CH_2)_2-$, $-(CH_2)_3-$, or $-(CH_2)_4-$. In general, compounds of formula (1) in which $-(CH_2)_n-$ is $-(CH_2)_2-$ or $-(CH_2)_3-$ are preferred. In a particular general preference $-(CH_2)_n-$ is $-(CH_2)_3-$.

In general compounds of formula (1) in which the chain $-(CH_2)_m-$ is $-CH_2-$ are preferred.

When $R^2$ in compounds of formula (1) represents a group $Ar(CH_2)_p$, p may represent an integer (1, 2 or 3, but is preferably zero. The group Ar may be for example phenyl or phenyl substituted by one, two or three substituents, particularly one substituent. It will be appreciated that when Ar is a substituted phenyl group the substituent(s) may be present in positions ortho, meta or para to the position linking the group to the rest of the molecule. In general, however, when just one substituent is present this is preferably in the para position.

Examples of the substituents which may be present on phenyl groups represented by Ar include fluorine, chlorine, bromine or iodine atoms or methyl, ethyl, methoxy, ethoxy, hydroxyl, trifluoromethyl, nitro or amino groups.

Particular examples of substituted phenyl groups represented by Ar include phenyl substituted by a bromine, chlorine or iodine atom, or a methyl, methoxy, hydroxyl, trifluoromethyl, nitro or amino group. In another example, Ar may be 2, 4, 6-trimethylphenyl.

When $R^2$ in the compounds of formula (1) is a naphthyl group it may be 1- or 2-naphthyl.

In one general preference the group $R^2$ in compounds of formula (1) is a phenyl group.

According to a further general preference, the group $R^2$ in compounds of formula (1) is a 2-naphthyl group.

Particularly useful compounds according to the invention are: [3-[3-[(phenylsulphonyl)amino]propyl]-phenoxy]acetic acid; [3-[3-[(2-naphthalenylsulphonyl)amino]propyl]phenoxy]acetic acid; and their physiologically acceptable salts.

Compounds of formula (1) inhibit blood platelet aggregation, bronchoconstriction and vasoconstriction. A test to determine inhibition of blood platelet aggregation is as described by Lumley and Humphrey (J. Pharmacol. Methods, 1981, 6, 153-166) using Collagen as the pro-aggregatory agent.

The ability of the compounds of the invention to inhibit vasoconstriction or bronchoconstriction is determined using the relevant isolated tissue (e.g. spirally cut rat aortic strip or guinea-pig lung parenchymal strip) by measuring the effect of the compound to be tested on the contraction of the tissue to [1R-[1α,4α,5β(Z),6α(1E,3S*)]]-7-[6-(3-hydroxy-1-octenyl)-2-oxabicyclo[2,2,1]hept-5-yl]-5- heptenoic acid (U-46619).

The compounds are thus of interest in the treatment of asthma, and in the treatment and prophylaxis of occlusive vascular disease, including myocardial infarction, cardiac fatalities, angina, transient ischaemic attacks and cerebral infarction, atherosclerosis and vessel wall disease, peripheral vascular disease, diabetic nephropathy and retinopathy, postoperative thrombosis and pulmonary embolism, and for use in renal dialysis. In addition, the compounds of the invention are considered to be useful in the prophylaxis of peri- and postoperative complications following organ transplantation (particularly cardiac and renal), coronary artery bypass, angioplasty, thrombolysis and endarterectomy.

The compounds are also of potential use in the treatment of adult respiratory distress syndrome and the prevention of relapse of healed peptic ulcers.

The compounds may be formulated in a conventional manner for use with one or more pharmaceutical carriers.

For oral administration, the pharmaceutical composition may take the form of, for example, tablets, capsules, powders, solutions, syrups or suspensions prepared by conventional means with acceptable excipients.

The compounds may be formulated for parenteral administration by continuous infusion. Formulations for injections may be presented in unit dosage form in ampoules, or in multi-dose containers, with an added preservative. The compositions may take such forms as suspensions, solutions or emulsions in oil or aqueous vehicles, and may contain formulatory agents such as suspending, stabilising and/or dispersing agents. Alternatively, the active ingredient may be in powder form for reconstitution before use with a suitable vehicle e.g. sterile pyrogen-free water.

For administration by inhalation the compounds are conveniently delivered in the form of an aerosol spray presentation from pressurised packs or a nebuliser, or as a cartridge from which the powdered composition may be inhaled with the aid of a suitable device. In the case of a pressurised aerosol the dosage unit may be determined by providing a valve to deliver a metered amount.

For use as antithrombotic agents, the compounds are preferably administered orally, for example in amounts of 0.05 to 10mg/kg body weight, 1 to 4 times daily, or intravenously, for example in amounts of 0.01 to 25 mg/kg body weight, 1 to 4 times daily.

For use in the treatment of asthma, the compounds may also be administered orally in amounts of 0.05 to 10mg/kg body weight, 1 to 4 times daily; preferably however they are administered by inhalation at doses varying from 0.02 to 30mg, preferably 0.02 to 3.0mg, 1 to 4 times daily. The compounds may be used in combination with other antiasthmatic agents.

The precise dose administered will of course depend on the age and condition of the patient.

Suitable methods for preparing compounds of formula (1) are described below, the groups $R^1$, $R^2$, $-(CH_2)_n-$ and $-(CH_2)_m-$ being as defined above except where otherwise indicated. It will be appreciated that the following reactions might require the use of, or conveniently might be applied to, starting materials having protected functional groups, and deprotection might thus be required as a final step to yield a compound of the invention. Protection and deprotection of functional groups may be effected using conventional techniques. Thus, for example, amino groups may be protected by acylation, subsequent deacylation being effected when desired by hydrolysis using for example an acid such as hydrochloric acid.

(a) Compounds of formula (1) may be prepared by alkylation of a phenol of formula (2)

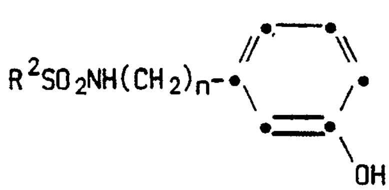

(2)

using an alkylating agent $L(CH_2)_mCOOR^1$ [where L is a leaving group, for example a halogen atom such as chlorine, bromine or iodine, or a hydrocarbylsulphonyloxy group such as methanesulphonyloxy or p-toluenesulphonyloxy] and followed where necessary by hydrolysis as described in section (c) below.

The alkylation is preferably effected in the presence of a base, for example an inorganic base such as potassium carbonate in a solvent such as an alcohol e.g. ethanol or an aqueous alcohol, e.g. aqueous ethanol, or sodium hydride in a solvent such as dimethylformamide. The reaction may be performed at a temperature up to and including reflux.

The alkylation is particularly suitable for the preparation of compounds of formula (1) wherein m is 1 and $R^2$ is other than phenyl substituted by a hydroxyl group.

The alkylating agents $L(CH_2)_m$ $COOR^1$ used in this process are either known compounds or may be prepared by methods analogous to those used for the preparation of the known compounds.

(b) In another process, compounds of formula (1) in which $R^1$ is $C_{1-4}$ alkyl may be prepared by sulphonylation of an amine of formula (3)

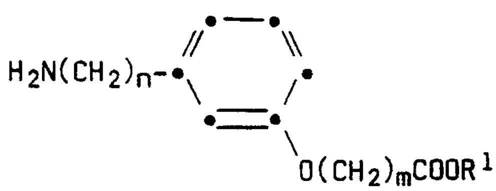

(3)

(wherein $R^1$ is $C_{1-4}$ alkyl) with a reactive derivative of a sulphonic acid $R^2SO_3H$ such as a sulphonyl halide for example a chloride $R^2SO_2Cl$.

The sulphonylation is preferably effected in the presence of a suitable acid scavenger, for example an organic base such as triethylamine or pyridine, either neat or in an appropriate solvent such as acetonitrile.

(c) Compounds of formula (1) in which $R^1$ is -H may be prepared by hydrolysis of a corresponding ester (e.g. in which $R^1$ is a $C_{1-4}$ alkyl).

The hydrolysis can in general be effected by conventional methods, under acidic or basic conditions, as appropriate, in a polar solvent such as water, or an alcohol e.g. methanol or ethanol, or ketone e.g. acetone, at any suitable temperature up to and including reflux. Suitable bases include inorganic bases (e.g. alkali metal hydroxides such as sodium hydroxide or potassium hydroxide). Alternatively the ester may be cleaved using an acid such as an inorganic acid e.g. hydrochloric acid.

(d) Compounds of formula (1) in which $R^1$ is $C_{1-4}$ alkyl may be prepared by esterification of the corresponding carboxylic acid (in which $R^1$ is -H).

Conventional esterification techniques may be used, for example by reaction with an appropriate alcohol in the presence of a mineral acid such as hydrochloric acid or sulphuric acid.

(e) In a further process, compounds of formula (1) in which $-(CH_2)_n-$ is $-(CH_2)_3-$ or $-(CH_2)_4-$ and $R^2$ is other than thienyl, phenyl substituted by nitro, or $\beta$-styryl may be prepared by reduction of a compound of formula (4)

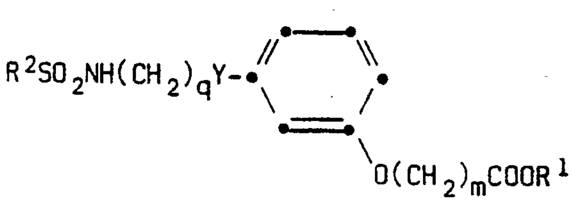

(4)

(wherein q is an integer 1 or 2, Y is -CH=CH- or -C≡C- and $R^2$ is as just defined) using for example hydrogen in the presence of a metal catalyst e.g. palladium on a support such as carbon in a suitable solvent such as an ester, e.g. ethyl acetate.

(f) Compounds of formula (1) in which $R^2$ is phenyl substituted by a hydroxyl group may be prepared by demethylation of a corresponding compound of formula (1) in which $R^2$ is phenyl substituted by a methoxy group. The reaction may be performed using for example boron tribromide in a solvent such as dichloromethane at a temperature of 0 °C to ambient.

(g) Where salts of compounds of the invention are desired, such salts may be formed by conventional

methods. For example salts of bases may be prepared by treatment of a compound of the invention with a base in a suitable solvent, e.g. ether or an alcohol such as ethanol. Salts of acids may be prepared by adding the acid (e.g. hydrogen chloride) to a solution of the compound of formula (1) in an organic solvent such as ether.

Salts may also be formed by conversion of one salt of a compound of the invention into another, e.g. by ion exchange using conventional methods.

The intermediate compounds of formula (2) may be prepared by demethylation of compounds of formula (5)

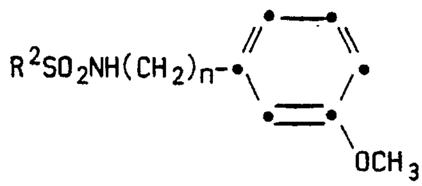

(5)

using for example boron tribromide as described in process (f) above.

The intermediates of formula (5) may be prepared by sulphonylation of amines of formula (6)

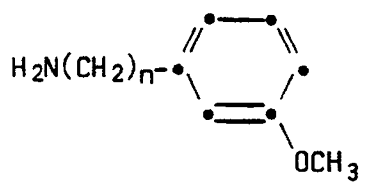

(6)

using an appropriate sulphonyl chloride $R^2SO_2Cl$ in the presence of an organic base such as triethylamine and an inert solvent such as acetonitrile.

The amines of formula (6) are either known compounds (see for example D.S. Kashdan et al, J. Org. Chem. 1982, 47, 2638) or may be prepared by methods analogous to those used for the preparation of the known compounds.

The intermediate amines of formula (3) may be prepared from the amines of formula (6) by the following sequence of reactions:

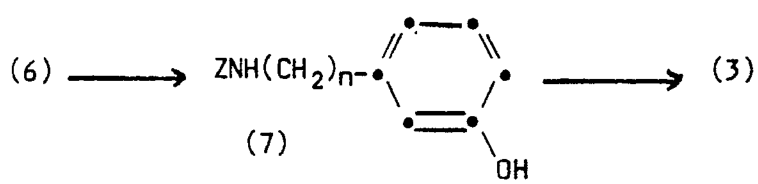

(6) (7) (3)

(wherein Z is a protecting group, for example an acetyl or benzoyl group).

Protection of an amine (6), for example by reaction with acetyl or benzoyl chloride, followed by demethylation (using e.g. boron tribromide as described above or a nucleophile such as $-SC_2H_5$) yields a phenol of formula (7). Alkylation of a phenol (7) with an alkylating agent $L(CH_2)_mCOOR^1$ as described in process (a) above yields a compound of formula (3) in which the amino function is protected. Subsequent deprotection, in accordance with conventional practice, followed, if necessary, by esterification to reform the carboxylic ester group yields a compound of formula (3).

Intermediate acids of formula (4) where $R^1$ is a hydrogen atom may be prepared by hydrolysis of the corresponding ester of formula (4), where $R^1$ is $C_{1-4}$ alkyl, using the techniques described in process (c) above.

Intermediate esters of formula (4) may be prepared by sulphonylation of an amine of formula (8)

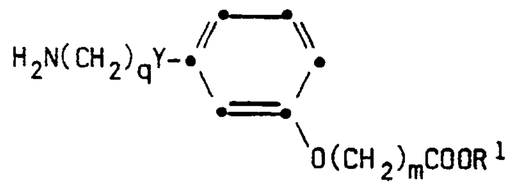

(8)

(wherein $R^1$ is a $C_{1-4}$ alkyl group) using the procedures described in process (b) above.

The amines of formula (8) may be prepared by reaction of a phenyl halide of formula (9)

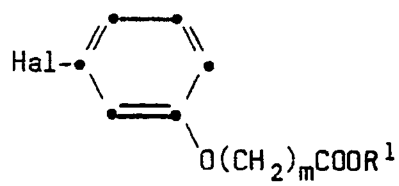

(9)

(wherein Hal is a halogen atom such as a bromine or iodine atom) with a protected amine $W(CH_2)_qYH$ (where W is a protected amino group such as phthalimido group) followed where necessary by removal of any protecting group. The reaction is performed in the presence of a metal catalyst (e.g. palladium acetate), a phosphine such as tri(o-tolyl)-phosphine or a complex of a metal catalyst and phosphine such as bis-(triphenylphosphino)palladium II chloride, and a base such as an organic amine e.g. triethylamine. In reactions of this type in which Y is -C≡C-, it is preferable to additionally include a copper halide, e.g. copper iodide.

Where desired, intermediate ester of formula (4) may be prepared directly using a halide of formula (9), and a sulphonamide $R^2SO_2NH(CH_2)_qYH$. Thus, for example, an intermediate ester of formula (4) in which $R^2$ is phenyl may be conveniently prepared using a halide of formula (9) and sulphonamide $R^2SO_2NH(CH_2)_qYH$ (where $R^2$ is phenyl) using the reaction conditions just described.

Halides of formula (9) may be prepared by alkylation of the corresponding known phenols using for example a method described in process (a) above.

The protected amines $W(CH_2)qYH$ and the sulphonamides $R^2SO_2NH(CH_2)_qYH$ are either known compounds or may be prepared by methods analogous to those used for the preparation of the known compounds.

The following compounds illustrate the invention.

Temperatures are in °C. "Dried" refers to drying with $MgSO_4$. Chromatography and thin layer chromatography (t.l.c.) refer to the use of silica gel. "Hyflo" is a filtration aid.

The following abbreviations are used ER-ether, EA-ethyl acetate, PE-petroleum ether (b.p. 40-60°), DMF-dimethylformamide, THF-tetrahydrofuran, NaH-sodium hydride dispersed in oil, $Et_3N$-triethylamine.

Intermediate 1

N-[2-(3-Hydroxyphenyl)ethyl]benzamide

A solution of N-[2[(3-methoxyphenyl)ethyl]benzamide (1g) in dry DMF (20mℓ) was added dropwise to a stirred suspension of 60% NaH (480mg) in DMF (20mℓ) containing ethanethiol (0.9mℓ) under nitrogen and the mixture was heated at 120° for 5h. The cooled mixture was diluted with water, adjusted to pH2 using 2N HCl and extracted with EA. The combined extracts were washed with water, dried and evaporated and the residue was crystallised from ethanol-ER-cyclohexane to give the title compound (0.69g). A recrystallised portion had m.p. 123.5-125°.

Intermediate 2

[3-[2-[(Benzoyl)amino]ethyl]phenoxy]acetic acid

A mixture of Intermediate 1 (230mg), ethylbromoacetate (0.12ml) and $K_2CO_3$ (238mg) in 19:1 ethanol-water (4ml) was heated at reflux under nitrogen for 20h. After allowing the mixture to cool, water was added

and the pH of the solution was adjusted to 2 with 2N HCl. The solution was extracted with ER and the combined extracts were extracted with 8% $NaHCO_3$. The combined $NaHCO_3$ extracts were adjusted to pH2 with 2N HCl and extracted with ER. The combined extracts were dried and evaporated to give a solid which was purified by crystallisation from ethanol-ER-cyclohexane to give the title compound (180mg) m.p. 141.5-142°

Intermediate 3

[3-(2-Aminoethyl)phenoxy]acetic acid, hydrochloride

Intermediate 2 (3.95g) was suspended in 6N HCl (125mℓ) and heated at 110° with stirring for 17h. The solution was cooled and extracted with EA (2 x 75mℓ). The aqueous layer was evaporated to dryness in vacuo to afford the title compound as a colourless solid (2.97g) m.p. 212-218°.

Intermediate 4

Methyl [3-(2-Aminoethyl)phenoxy]acetate, hydrochloride

Acetyl chloride (5mℓ) was added dropwise to stirred methanol (50mℓ) under nitrogen. After the mixture had cooled to ambient temperature, Intermediate 3 (1.049g) was added and stirring continued for 6h. Evaporation of the solvent in vacuo gave a solid which was triturated with ER to give the title compound as a powder (1.052g). A portion was crystallised from EA-methanol m.p. 117-122°

Intermediate 5

5a) Methyl 3-Iodophenoxyacetate

A vigorously stirred mixture of 3-iodophenol (14.7g), $K_2CO_3$ (12.92g) and methylbromoacetate (7.6mℓ) in DMF (100mℓ) was kept at 20° for 2h. The mixture was diluted with EA (250mℓ) and washed with water (3x100mℓ), 2N HCl (100mℓ) dried and evaporated. The residue was triturated with PE to give the title compound (16.25g) m.p. 50-52°. The following compound was prepared in a similar manner:-

5(b) Methyl 3-Bromophenoxyacetate, m.p. 39-42° from 3-bromophenol.

Purification by distillation b.p. 130°/0.3torr. (air bath temperature).

Intermediate 6

Methyl 2-(3-Iodophenoxy)propionate

A stirred mixture of 3-iodophenol (4.25g), methyl acrylate (10mℓ) and benzyltriethylammonium hydroxide (0.5mℓ; 40% solution in methanol) was heated under reflux for 40h. Fresh benzyltriethylammonium hydroxide (0.5mℓ) was added and heating continued for a further 24h. The excess of methyl acrylate was removed in vacuo and the residue in $CH_2Cl_2$ (50mℓ) was washed with water (2x20mℓ), dried and evaporated. The residue was purified by chromatography on silica deactivated with $Et_3N$ eluting with 1:1 PE-ER to give the title compound as an oil (2.72g). I.r. ($CHBr_3$) 1730cm$^{-1}$.

Intermediate 7

N-(2-Propynyl)benzenesulphonamide

Benzenesulphonyl chloride (5.6mℓ) in $CHCl_3$ (10mℓ) was added to a cooled (5°), stirred slurry of propargylamine hydrochloride (2.676g) in pyridine (20mℓ) under nitrogen. The resulting solution was stirred at ambient temperature for 18h then diluted with EA (150mℓ), washed with 2N HCl (2x75mℓ) and 8% $NaHCO_3$ (75mℓ), dried and evaporated. The title compound was obtained as an oil which slowly solidified on storage (5.196g) m.p. 52-57°.

Intermediate 8

8a) (E)-Methyl [3-[3-(1,3-Dihydro-1,3-dioxo-2H-isoindol-2-yl)-1-propenyl]phenoxy]acetate

A solution of 1,3-Dihydro-1,3-dioxo-2-(2-propenyl)-2H-isoindole (7.6g), Intermediate 5a (11.87g), palladium acetate (84mg) and tri(o-tolyl)phosphine (244mg) in $CH_3CN$ (18mℓ) and $Et_3N$ (18mℓ) was heated under reflux for 2h. The cooled mixture in $CHCl_3$ (300mℓ) was washed with 2N HCl (2x150mℓ), 8% $NaHCO_3$ (150mℓ) and brine (100mℓ). The organic layer was dried and evaporated and the residue was triturated with ER-PE to give the title compound (12.1g) m.p. 127-130°.

The following compounds were prepared in a similar manner:-

8b) Methyl [3-[3-[(Phenylsulphonyl)amino]-1-propynyl]phenoxy]acetate, m.p. 56-60°, from Intermediates 5b and 7. Purification by chromatography using 3:1 ER-PE as eluant followed by chromatography on silica deactivated with $Et_3N$ eluting with 3:1 ER-PE.

8c) (E)-Methyl 2-[3-[3-[(Phenylsulphonyl)amino]-1-propenyl]phenoxy]-propionate, from Intermediate 6 and N-(2-propenyl)benzenesulphonamide. Purification by chromatography using 4:1 ER-PE as eluant. I.r. (film) 3280, 1735, 1328, 1160, 970cm$^{-1}$.

8d) (E)-Methyl [3-[4-[Phenylsulphonyl)amino]-1-butenyl]phenoxy]-acetate, from Intermediate 5a and N-(3-butenyl)benzenesulphonamide. Purification by chromatography using 3:2 ER-PE as eluant. I.r. (film) 3280, 1750, 1325, 1160, 970cm$^{-1}$.

## Intermediate 9

(E)-Methyl [3-(3-Amino-1-propenyl)phenoxy]acetate, hydrochloride

Hydrazine hydrate (2.2g) was added to a hot (60°) stirred suspension of Intermediate 8a (11.76g) in methanol (500mℓ). The mixture was allowed to cool to ambient temperature over 19h then acidified with concentrated HCl and evaporated to dryness. The residue was stirred with water (500mℓ), filtered and the filtrate was evaporated to dryness. The residue in methanol (100mℓ) containing acetyl chloride (4mℓ) was kept at ambient temperature for 24h. The mixture was filtered and evaporated in vacuo to give the title compound (4.6g). A portion crystallised from isopropanol had m.p. 145-155°.

## Intermediate 10

10a) N-[2-(3-Methoxyphenyl)ethyl]benzenesulphonamide

A solution of benzenesulphonyl chloride (12.76mℓ) in dry $CH_3CN$ (10mℓ) was added to a solution of 3-methoxy(2-phenethylamine) (15.12g) and $Et_3N$ (14.04mℓ) in dry $CH_3CN$ (90mℓ) at 20°. After stirring the resulting suspension for 4h, some of the $CH_3CN$ (~50mℓ) was removed by evaporation. ER (400mℓ) was added and the mixture washed successively with 2N HCl (2x50mℓ) and brine (2x50mℓ). The ethereal solution was dried and evaporated to give an oil which was purified by chromatography eluting with 1:1 PE-ER to provide the title compound as an oil (25.88g).

Analysis Found: C,61.9; H,5.9; N,4.8.

$C_{15}H_{17}NO_3S$ requires C,61.8; H,5.9; N,4.8%.

The following compounds were prepared in a similar manner:-

10b) Methyl [3-[2-[[[4-Acetylamino)phenyl]sulphonyl]amino]ethyl]-phenoxy]acetate, m.p. 117-127° from Intermediate 4 and 4-(Acetylamino)benzenesulphonyl chloride in pyridine. Purification by chromatography using EA as eluant.

10c) N-[3-Methoxyphenyl)methyl]benzenesulphonamide, m.p. 76.5-78° from 3-methoxybenzylamine and benzenesulphonyl chloride. Purification by chromatography using 1:1 PE-ER followed by ER as eluants and then by crystallisation from EA-PE.

10d) N-[3-Methoxyphenyl)methyl]-4-(trifluoromethyl)benzenesulphonamide, m.p. 89-91°, from 3-methoxybenzylamine and 4-(trifluoromethyl)benzenesulphonyl chloride.

10e) (E)-Methyl [3-[3-[[[4-(Trifluoromethyl)phenyl]sulphonyl]amino]-1-propenyl]phenoxy]acetate, m.p. 90-92° from Intermediate 9 and 4-(trifluoromethyl)benzenesulphonyl chloride. Purification by chromatography using 2:1 ER-PE as eluant.

10f) (E)-Methyl [3-[3-[[(1-Naphthalenyl)sulphonyl]amino]-1-propenyl]phenoxy]acetate, from Intermediate 9 and 1-naphthalene-sulphonyl chloride. Purification by chromatography using 2:1 ER-PE as eluant. T.l.c. 2:1 ER-PE Rf 0.3.

10g) (E)-Methyl [3-[3-[[(2-Naphthalenyl)sulphonyl]amino]-1-propenyl]phenoxy]acetate, m.p. 78.5-79.5°, from Intermediate 9 and 2-naphthalenesulphonyl chloride. Purification by chromatography using 2:1 ER-PE as eluant.

10h) (E)-Methyl [3-[3-[[4-Chlorophenyl)sulphonyl]amino]-1-propenyl]phenoxy]acetate, from Intermediate 9 and 4-chlorobenzene-sulphonyl chloride. Purification by chromatography using 3:2 ER-PE as eluant. T.l.c. ER Rf 0.42

Intermediate 11

11a) N-[2-(3-Hydroxyphenyl)ethyl]benzenesulphonamide

A solution of $BBr_3$ (25g) in dry $CH_2Cl_2$ (80mℓ) was added dropwise to a cold (5°) solution of Intermediate 10a (14.45g) in dry $CH_2Cl_2$ (200mℓ). The cooling bath was removed and the solution stirred for 2h. Methanol (200mℓ) was added cautiously and after a further 1h the solvents were evaporated and the residue was purified by chromatography eluting with ER to give the title compound as an oil (14.01g).

Analysis Found: C,60.3; H,5.4; N,5.0.

$C_{14}H_{15}NO_3S$ requires C,60.6; H,5.45; N,5.05%.

The following compounds were prepared in a similar manner:-

11b) N-[3-Hydroxyphenyl)methyl]benzenesulphonamide, m.p. 107-110° from Intermediate 10c. Purification by chromatography using 1:1 PE-ER followed by ER as eluants.

11c) N-[(3-Hydroxyphenyl)methyl]-4-(trifluoromethyl)benzenesulphonamide, m.p. 132-134°, from Intermediate 10d. Purification by chromatography using ER as eluant.

Intermediate 12

12a) (E)-[3-[3-[[(1-Naphthalenyl)sulphonyl]amino]-1-propenyl]phenoxy]-acetic acid

A solution of Intermediate 10f (0.7g) in methanol (11.5mℓ), THF (5mℓ) and 5N NaOH (11.5mℓ) was kept at ambient temperature for 3h. The mixture was diluted with water (150mℓ) and extracted with ER (150mℓ). The aqueous layer was diluted with 2N HCl (100mℓ) and extracted with $CH_2Cl_2$ (2x50mℓ). The combined extracts were dried and evaporated to give the title compound (0.37g) m.p. 101.5-102.5°.

The following compounds were prepared in a similar manner:-

12b) (E)-[3-[3-[[(2-Naphthalenyl)sulphonyl]amino]-1-propenyl]phenoxyacetic acid, m.p. 121-122°, from Intermediate 10g.

12c) (E)-2-[3-[3-[(Phenylsulphonyl)amino]-1-propenyl]phenoxy]propionic acid, m.p. 117-121°, from Intermediate 8c using 5N NaOH and ethanol. Purification by chromatography using ER followed by 99:1 ER-AcOH as eluants.

12d) (E)-[3-[4-(Phenylsulphonyl)amino]-1-butenyl]phenoxy]acetic acid, dicyclohexylamine salt, m.p. 139-143°, from Intermediate 8d using KOH in methanol-water. A portion of the acid was converted into the dicyclohexylamine salt in ER.

Intermediate 13

(E)-Methyl [3-[3-[Phenylsulphonyl)amino]-1-propenyl]phenoxy]acetate

A solution of N-(2-propenyl)benzenesulphonamide (19.3g), Intermediate 5a (28.0g), palladium acetate (217mg), and tri(o-tolyl) phosphine (620mg) in $CH_3CN$ (30mℓ) and $Et_3N$ (30mℓ) was heated at reflux under nitrogen for 3.5h. The mixture was cooled, filtered through hyflo and evaporated. The residue was redissolved in $CH_2Cl_2$ (300mℓ) and washed with 2M HCl (2x75mℓ), then 8% $NaHCO_3$ (75mℓ), dried and evaporated to give an orange gum (36.0g), a portion (2.1g) of this gum was purified by chromatography on silica eluting with 9:1 $CH_2Cl_2$-ER to give the title compound as a pale yellow solid (950mg).

Analysis Found: C,59.7; H,5.2; N,3.8.

$C_{18}H_{19}NO_5S$ requires C,59.8; H,5.3; N,3.9%.

Intermediate 14

2-[N-(2-Propynyl)]naphthalenesulphonamide

A solution of 2-naphthalenesulphonyl chloride in $CH_2Cl_2$ (30mℓ) was added dropwise to a cooled (0°C), stirred solution of propargylamine (3.7mℓ) in dry pyridine (20mℓ) under nitrogen. The mixture was stirred for 1h, then diluted with $CH_2Cl_2$ (70mℓ) and washed with 2M HCl (2x125mℓ), then 8% $NaHCO_3$ (75mℓ), dried and evaporated to give the title compound as a pale brown solid (10.1g).

Analysis Found: C,63.65; H,4.5; N,5.4.

$C_{13}H_{11}NO_2S$ requires C,63.65; H,4.5; N,5.7%.

Intermediate 15

Methyl [3-[3-[(2-Naphthalenylsulphonyl)amino]-1-propynyl]phenoxy]-acetate

A solution of Intermediate 14 (600mg), Intermediate 5a (550mg), bis(triphenylphosphine) palladium dichloride, and copper (I) iodide in dry THF (1mℓ) and diethylamine (6mℓ) was stirred at room temperature under nitrogen for 18h then poured into 2M HCl (30mℓ) and extracted with $CH_2Cl_2$ (3x20mℓ). The combined organic extracts were washed with 2M HCl (10mℓ) and then 8% $NaHCO_3$ (10mℓ), dried, and evaporated to give an orange gum which was purified by chromatography on silica eluting with 2:1 hexane-EA, then 1:1 hexane-EA to give the title compound as a pale brown solid (495mg).
Analysis Found: C,64.5; H,4.7; N,3.5.
$C_{22}H_{19}NO_5S$ requires C,64.5; H,4.7; N,3.4%.

Example 1

1a) [3-[2-[(Phenylsulphonyl)amino]ethyl]phenoxy]acetic acid

A mixture of Intermediate 11a (4.1g), ethylbromoacetate (1.8mℓ) and $K_2CO_3$ (3.7g) in 19:1 ethanol-water (60mℓ) was heated at reflux under nitrogen for 20h. After allowing the mixture to cool, the solvent was evaporated, water (20mℓ) was added and the pH of the solution was adjusted to 2 with 2N HCl. The solution was extracted with ER (3x50mℓ) and the combined extracts were extracted with 8% $NaHCO_3$ - (3x20mℓ). The combined $NaHCO_3$ extracts were adjusted to pH2 with 2N HCl and extracted with ER (3x40mℓ). The combined extracts were dried and evaporated to give an oil which was purified by chromatography eluting with 40:10:1 ER-PE-AcOH to give an oil (3.04g). A portion of the oil was dissolved in EA-toluene, decolourised with charcoal and the solvents removed in vacuo. The residue was triturated with ER to give the title compound as a powder m.p. 92-95°.
Analysis Found: C,57.3; H,5.15; N,4.1.
$C_{16}H_{17}NO_5S$ requires C,57.3; H,5.1; N,4.2%.

The following compound was prepared in a similar manner:-

1b) [3-[[(Phenylsulphonyl)amino]methyl]phenoxy]acetic acid, m.p. 128-131° from Intermediate 11b. Purification initially by chromatography using 10:5:1 PE-EA-AcOH as eluant and then by crystallisation from EA.
Analysis Found: C,55.9; H,4.7; N,4.4.
$C_{15}H_{15}NO_5S$ requires C,56.1; H,4.7; N,4.4%.

Example 2

Ethyl [3-[2-[(Phenylsulphonyl)amino]ethyl]phenoxy]acetate

A solution of the product of Example 1a (0.216g) in ethanol (6mℓ) containing conc. $H_2SO_4$ (0.2mℓ) was kept at ambient temperature for 65h. The solution was diluted with 2N $Na_2CO_3$ to pH7 and extracted with EA (2x10mℓ). The combined extracts were dried and evaporated and the residue was purified by chromatography eluting with 1:1 PE-ER to give the title compound as an oil (0.184g).
I.r. (film) 3280, 1750, 1325, 1160cm$^{-1}$
Analysis Found: C,59.9; H,5.8; N,4.0.
$C_{18}H_{21}NO_5S$ requires C,59.5; H,5.8; N,3.85%.

Example 3

Methyl [3-[[[[4-(Trifluoromethyl)phenyl]sulphonyl]amino]methyl]-phenoxy]acetate

A solution of Intermediate 11c (344mg) in dry DMF (8mℓ) was stirred under nitrogen for 1.5h with 80% NaH (78mg). Methylbromoacetate (175mg) in DMF (8mℓ) was added dropwise over 30 min. and the mixture was stirred at ambient temperature for 2h. The mixture was diluted with pH 6.5 phosphate buffer (100mℓ) and extracted with EA (4x50mℓ). The combined extracts were washed with brine, dried and evaporated to give an oil which was purified by chromatography on silica, deactivated with $Et_3N$ eluting with ER to give the title compound (132mg), m.p. 83-85°
Analysis Found: C,50.8; H,3.9; N,3.4.
$C_{17}H_{16}F_3NO_5S$ requires C,50.6; H,4.0; N,3.5%.

Examples 4-18

Methyl [3-[2-[(Sulphonyl)amino]ethyl]phenoxy]acetates

Table 1 summarises the preparation of the title compounds by the following method.
A mixture of Intermediate 4 and an excess of the sulphonyl chloride was stirred in pyridine under nitrogen until reaction was complete as judged by t.l.c. The mixture was diluted with 2N HCl at 0° and extracted with EA. The extracts were washed with water, dried and evaporated and the residue was purified by chromatography as indicated in the Table.

Table 1

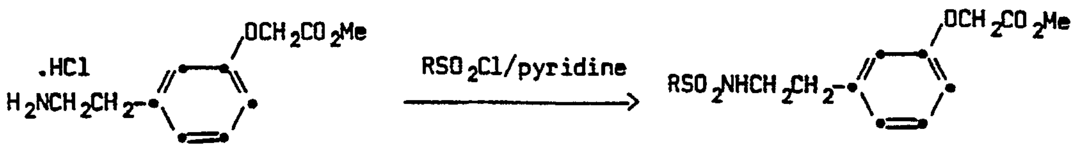


Intermediate 4 Examples 4-18

| Example No | R | Chromatography system (t.l.c. Rf) | m.p. °C | I.r. (film) $cm^{-1}$ |
|---|---|---|---|---|
| 4 | Ph | ER (0.36) | 94-97 | (Nujol) 3260, 1748, 1318, 1162 |
| 5 | 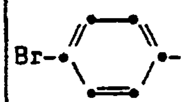 | 1:1 PE-ER then ER (0.48) | - | 3290, 1760, 1330, 1160 |
| 6 | 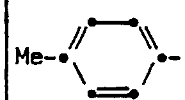 | ER (0.54) | - | 3280, 1760, 1325, 1160 |
| 7 | 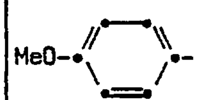 | ER+ (0.21) | - | 3280, 1755, 1335, 1150 |
| 8 | 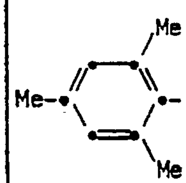 | 2:1 ER-PE (0.26) | 80-82 | (Nujol) 3280, 1760, 1323, 1160 |
| 9 | 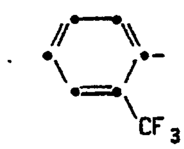 | ER+ (0.27) | - | 3320, 1755, 1342, 1160 |
| 10 | 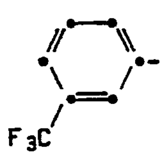 | ER+ (0.31) | - | 3280, 1750, 1325, 1160 (br.) |
| 11 | 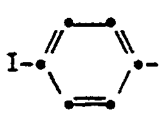 | 2:1 ER-PE (0.17) | 71-74 | (Nujol) 3260, 1768, 1320, 1153 |
| 12 | 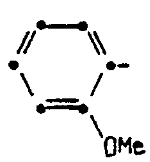 | ER (0.23) | 83-85 | (Nujol) 3275, 1753, 1320, 1160 |

Table 1 (cont.)

| Example No | R | Chromatography system (t.l.c. Rf) | m.p. °C | I.r. (film) $cm^{-1}$ |
|---|---|---|---|---|
| 13 | $PhCH_2CH_2$- | 2:1 ER-PE (0.25) then ER | - | 3290, 1750, 1330, 1140 |
| 14 | 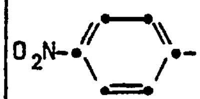 | ER+ (0.28) | 96-99 | ($CHBr_3$) 3365, 1755, 1530, 1350 |
| 15 | 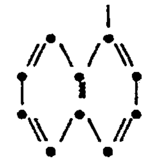 | 1:1 $CH_2Cl_2$-ER then ER (0.61) | - | 3300, 1700, 1320, 1160 |
| 16 | 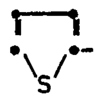 | ER+ (0.29) | - | 3280, 1755, 1340, 1130 |
| 17 | 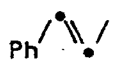 | ER+ (0.28) | - | 3270, 1715, 1320, 1150, 973 |
| 18 | 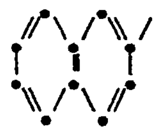 | 2:1 ER-PE then ER (0.42) | 94-96 | (Nujol) 3250, 1745, 1318, 1158 |

+ Silica deactivated with $Et_3N$

Example 19

Methyl [3-[2-[[(2-Hydroxyphenyl)sulphonyl]amino]ethyl]phenoxy]acetate

$BBr_3$ (1.2mℓ), 1M in $CH_2Cl_2$) was added to a stirred solution of the product of Example 12 (463mg) in $CH_2Cl_2$ (4mℓ) at 0° under nitrogen. After 16h at ambient temperature methanol (0.7mℓ) was cautiously added and stirring continued for 0.25h. The mixture was diluted with 8% $NaHCO_3$ and extracted with EA (3x40mℓ). The combined extracts were dried and evaporated and the residue was purified by chromatography using ER as eluant to give the title compound as an oil (302mg). T.l.c. ER Rf 0.41, I.r. (film) 3305 (br.), 1750, 1320, 1157$cm^{-1}$

Example 20

20a) Methyl [3-[3-[(Phenylsulphonyl)amino]propyl]phenoxy]acetate

A solution of Intermediate 8b (0.3g) in EA (10mℓ) was hydrogenated over 5% Pd/C (50mg) until uptake of hydrogen ceased. The catalyst was removed by filtration (hyflo) and the filtrate evaporated to give the title compound as an oil (0.298g).

T.l.c. ER Rf 0.38, I.r. (film) 3280, 1758, 1325, 1160$cm^{-1}$.

The following compounds were prepared in a similar manner:-

20b) Methyl [3-[3-[[[4-(Trifluoromethyl)phenyl]sulphonyl]amino]-propyl]phenoxy]acetate, m.p. 69-71°, from Intermediate 10e. Purification by trituration with 3:1 PE-ER. I.r. (Nujol) 3255, 1746, 1322, 1162$cm^{-1}$

20c) [3-[3-[(1-Naphthalenylsulphonyl)amino]propyl]phenoxy]acetic acid, from Intermediate 12a, t.l.c. 99:1 ER-AcOH Rf 0.33 I.r. (Film) 3290, 1735(br.), 1318, 1160$cm^{-1}$

20d) Methyl [3-[3-[[4-Chlorophenyl)sulphonyl]amino]propyl]phenoxy]-acetate, from Intermediate 10h, t.l.c. ER Rf 0.41, I.r. (Film 3275, 1750, 1330, 1160cm$^{-1}$

20e) 2-[3-[3-[(Phenylsulphonyl)amino]propyl]phenoxy]propionic acid, m.p. 70-74°, from Intermediate 12c, I.r. (Nujol) 3280, 1710, 1690, 1330, 1160cm$^{-1}$

20f Methyl [3-[4-[(Phenylsulphonyl)amino]butyl]phenoxy]acetate,from Intermediate 8d, t.l.c. 1:1 ER-PE Rf 0.09, I.r. (film) 3280, 1758, 1325, 1160cm$^{-1}$

20g) [3-[4-[(Phenylsulphonyl)amino]butyl]phenoxy]acetic acid, dicyclohexylamine salt, m.p. 112-116°, from Intermediate 12d. Purification by conversion into the dicyclohexylamine salt in ER. I.r. (Nujol) 3500-2300, 1630(br.), 1328, 1160cm$^{-1}$

Examples 21-35

[3-[2-[(Sulphonyl)amino]ethyl]phenoxy]acetic acids

Table 2 summarises the preparation of the title compounds by the following method:
Each ester (prepared as in Examples 4-19) was dissolved in aqueous NaOH, with the addition of an alcohol, e.g. methanol, if necessary, and allowed to stand at room temperature until hydrolysis was complete as judged by t.l.c. The solution was poured into an excess of 2N HCl and extracted with EA or $CH_2Cl_2$. The extracts were washed with water, dried and evaporated. The majority of compounds were obtained analytically pure by this method. If necessary a compound was further purified as indicated in the Table.

Table 2

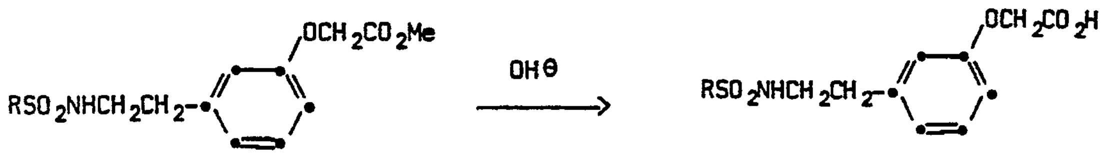


Examples 4-19 Examples 21-35

| Example No | R | m.p. °C | Analysis % Found | Analysis % Required | Purification Method |
|---|---|---|---|---|---|
| 21 | 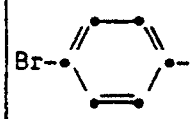 | 121-122.5 | C,46.5;H,3.8; N,3.3 | C,46.4;H,3.9; N,3.4 | - |
| 22 | 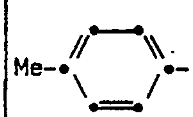 | 118-122 | C,58.4;H,5.4; N,3.9 | C,58.4;H,5.5; N,4.0 | Crystal-lisation from EA-PE |
| 23 | 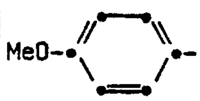 | 59-62 | C,55.9;H,5.3; N,3.7 | C,55.9;H,5.2; N,3.8 | - |
| 24 | 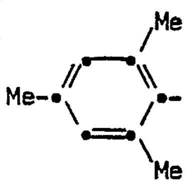 | 154-155 | C,60.5;H,6.25; N,3.7 | C,60.5;H,6.1; N,3.7 | - |
| 25 | 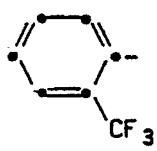 | 128-130 | C,50.0;H,3.8; N,3.3* | C,50.6;H,4.0; N,3.5 | Crystallis-ation from $CH_2Cl_2$-PE |
| 26 | 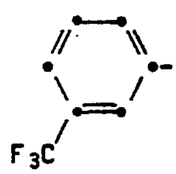 | 109-112 | C,50.5;H,4.0; N,3.4 | C,50.6;H,4.0; N,3.5 | - |
| 27 | 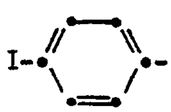 | 118-120 | C,41.7;H,3.5; N,3.0 | C,41.7;H,3.5; N,3.0 | - |
| 28 | 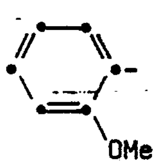 | 93-95 | C,55.8;H,5.2; N,3.8 | C,55.9;H,5.2; N,3.8 | - |

Table 2 (cont.)

| Example No | R | m.p. °C | Analysis % Found | Analysis % Required | Purification Method |
|---|---|---|---|---|---|
| 29 | $PhCH_2CH_2$- | 116-117 | C,59.5;H,5.9; N,3.9 | C,59.5;H,5.8; N,3.85 | Chromatography using 10:5:1 PE-EA-AcOH |
| 30 | 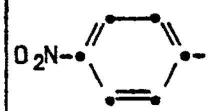 | 138-141 | C,50.6;H,4.2; N,7.1 | C,50.5;H,4.2; N,7.4 | - |
| 31 | 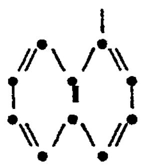 | 107.5-109.5 | C,62.2;H,5.0; N,3.6 | C,62.3;H,5.0; N,3.6 | - |
| 32 | 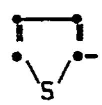 | 106-109 | C,48.6;H,4.2; N,3.9* | C,49.3;H,4.4; N,4.1 | - |
| 33 | 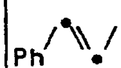 | 132-134 | C,59.0;H,5.45; N,3.7* | C,59.8;H,5.3; N,3.9 | - |
| 34 | 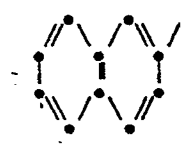 | 174-176 | C,61.9;H,5.2; N,3.4 | C,62.3;H,5.0; N,3.6 | - |
| 35 | 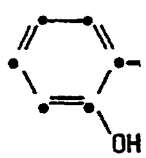 | 94-97 | C,55.0;H,5.0; N,3.7 | C,54.5;H,5.2; N,3.5 | Chromatography using ER then 199:1 ER-AcOH |

* Sample contains a trace of $CH_2Cl_2$ by n.m.r. spectroscopy

Example 36

The following compounds were prepared according to the procedure described for the compounds of Examples 21-35 in Table 2.

36a) [3-[[[[4-(Trifluoromethyl)phenyl]sulphonyl]amino]methyl]-phenoxy]acetic acid, m.p. 143-146° from the product of Example 3. Analysis Found: C,49.3; H,3.6; N,3.4. $C_{16}H_{14}F_3NO_5S$ requires C,49.4; H,3.6; N,3.6%.

36b) [3-[3-[[[4-(Trifluoromethyl)phenyl]sulphonyl]amino]propyl]-phenoxy]acetic acid, m.p. 122-125° from the product of Example 20b. Analysis Found: C,51.4; H,4.2; N,3.4. $C_{18}H_{18}F_3NO_5S$ requires C,51.8; H,4.35; N,3.4%.

36c) [3-[3-[[(4-Chlorophenyl)sulphonyl]amino]propyl]phenoxy]acetic acid, m.p. 106-110°, from the product of Example 20d. Purification by crystallisation from EA-PE.
I.r. (Nujol) 3260, 1710, 1700, 1325, 1160cm$^{-1}$

Example 37

Methyl [3-[2-[[(4-Aminophenyl)sulphonyl]amino]ethyl]phenoxy]acetate

A solution of Intermediate 10b (552mg) in methanol (10mℓ) and 2N HCl (10mℓ) was heated under reflux for 6h. The cooled mixture was adjusted to pH8 using 8% $NaHCO_3$ and extracted with EA (4x30mℓ). The combined extracts were dried and evaporated and the residue was purified initially by chromatography using 99:1 ER-AcOH as eluant followed by crystallisation from EA-PE to give the title compound (246mg) m.p. 82-84°.
Analysis Found: C,55.9; H,5.5; N,7.5.
$C_{17}H_{20}N_2O_5S$ requires C,56.0; H,5.5; N,7.7%.

Example 38

[3-[2-[[(4-Aminophenyl)sulphonyl]amino]ethyl]phenoxy]acetic acid

The bicarbonate layer described in the preparation of the compounds of Example 37 was adjusted to pH2 using 2N HCl and extracted thoroughly with EA. The combined extracts were dried and evaporated and the residue was crystallised from EA to give the title compound (94mg) m.p 149-152°.
Analysis Found: C,54.8; H,5.2; N,7.9.
$C_{16}H_{18}N_2O_5S$ requires C,54.9; H,5.2; N,8.0%.

Example 39

[3-[3-[(Phenylsulphonyl)amino]propyl]phenoxy]acetic acid

A mixture of the product of Example 20a (185mg) and potassium hydroxide (200mg) in methanol and water (1:1, 6mℓ) was stirred at 20° for 4h. The solution was diluted with water (40mℓ), washed with ER (25mℓ), acidified with 2N HCl and extracted with $CH_2Cl_2$ (2 x 30mℓ). The organic extracts were dried and evaporated to give a solid (140mg) which on crystallisation from EA-PE gave the title compound (131mg) as a white solid with m.p 100-103°.
Analysis Found: C,57.9; H,5.5; N,3.95
$C_{17}H_{19}NO_5S$ requires C,58.4; H,5.5; N,4.0%.

Example 40

[3-[3-[(2-Naphthalenylsulphonyl)amino]propyl]phenoxy]acetic acid

A solution of Intermediate 12b (0.3g) in ethyl acetate (30mℓ), was reduced by hydrogenation over a pre-reduced 10% palladium on charcoal catalyst (0.1g), The catalyst was filtered off through 'hyflo' and the filtrate evaporated in vacuo to give the title compound as a fawn solid (0.30g) m.p. 95-97°. I.r. (Nujol) 3700-2500 (br), 1715 (br), 1315, 1160cm$^{-1}$

Example 41

Methyl [3-[3-[(Phenylsulphonyl)amino]propyl]phenoxy]acetate

A solution of Intermediate 13 (935mg) in methanol (85mℓ) was hydrogenated over 10% Pd/C (350mg) until uptake of hydrogen ceased. The catalyst was removed by filtration (hyflo) and the filtrate was evaporated to give a yellow oil (980mg) which was purified by chromatography on silica eluting with 2:1 hexane-EA to give the title compound as a colourless oil (740mg), t.l.c. ER Rf 0.38
Analysis Found: C,59.5; H,6.05; N,4.0.
$C_{18}H_{21}NO_5S$ requires C,59.5; H,5.8; N,3.85%.

Example 42

Methyl [3-[3-[(2-Naphthalenylsulphonyl)amino]propyl]phenoxy]acetate

A solution of Intermediate 15 (470mg) in methanol (35mℓ) was hydrogenated over 10% Pd/C (200mg) until uptake of hydrogen ceased. The catalyst was removed by filtration (hyflo) and the filtrate was evaporated to give a yellow solid (400mg) which was purified by chromatography on silica eluting with 3:2 hexane-EA to give the title compound as a pale yellow crystalline solid (330mg) m.p. 78°. I.r. (Nujol) $\nu_{max}$ 1760; 3280cm$^{-1}$

Example 43

[3-[3-[(2-Naphthalenylsulphonyl)amino]propyl]phenoxy]acetic acid

A solution of Example 42 (326mg) in methanol (3mℓ) and 2M NaOH (18mℓ) was heated at reflux for 2.5h, then cooled and the methanol evaporated in vacuo. The resulting aqueous suspension was treated with 5M HCl (9mℓ) and extracted with EA (4x20mℓ). The combined extracts were dried and evaporated to give the title compound as a white solid (300mg) m.p. 118-121°.
Analysis Found: C,63.3; H,5.3; N,3.5.
$C_{21}H_{22}NO_5S$ requires C,63.0; H,5.5; N,3.5%.

The following is an example of a pharmaceutical formulation according to the invention. The term 'active ingredient' used in the example means a compound of formula (1) and may be a compound of the Examples, especially the compound of Example 39 or Example 40:

Tablets - Direct Compression

| | mg/tablet |
|---|---|
| Active Ingredient | 100.00 |
| Microcrystalline Cellulose B.P.C. | 298.00 |
| Magnesium Stearate | 2.00 |
| Compression Weight | 400.00 |

The active ingredient is sieved through a 250µm sieve, blended with the excipients and compressed using 10mm punches.

**Claims**

**Claims for the following Contracting States : BE, CH, DE, FR, GB, IT, LI, LU, NL, SE**

1. Compounds of the general formula (1)

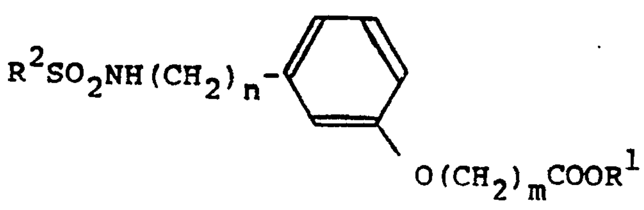

(1)

wherein

R[1] is a hydrogen atom or a $C_{1-4}$ alkyl group;

R[2] is a naphthyl, thienyl or β-styryl group

or a group $Ar(CH_2)p$- where p is zero or an integer from 1 to 3 and Ar is phenyl or phenyl substituted by one or more halogen atoms or $C_{1-4}$ alkyl, $C_{1-4}$ alkoxy, hydroxyl, trifluoromethyl, nitro or amino groups;

m is an integer 1 or 2;

n is an integer from 1 to 4;

and the physiologically acceptable salts thereof.

**2.** Compounds according to claim 1 in which $R^1$ is a hydrogen atom or a methyl group.

**3.** Compounds according to claim 1 or claim 2 in which m is 1.

**4.** Compounds according to any preceding claim in which n is 2 or 3.

**5.** Compounds according to any preceding claim in which $R^2$ is a naphthyl, phenyl or substituted phenyl group.

**6.** Compounds according to any of claims 1 to 4 in which $R^2$ is a 2-naphthyl or phenyl group.

**7.** A compound according to claim 1 said compound being [3-[3-[(phenylsulphonyl)amino]propyl]-phenoxy]acetic acid or a physiologically acceptable salt thereof.

**8.** A compound according to claim 1, said compound being [3-[3-[(2-naphthalenylsulphonyl)amino]propyl]-phenoxy]acetic acid or a physiologically acceptable salt thereof.

**9.** A phamaceutical composition comprising a compound according to any preceding claim together with one or more pharmaceutical carriers.

**10.** A process for the preparation of a compound according to claim 1 which comprises:
(a) alkylation of a compound of formula (2)

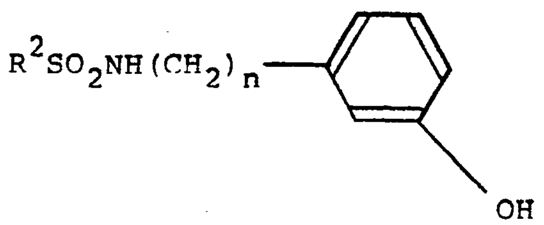

(2)

using an alkylating agent $L(CH_2)_mCOOR^1$ (where L is a leaving group);
(b) in the preparation of a compound in which $R^1$is a $C_{1-4}$ alkyl group, sulphonylating a compound of formula (3)

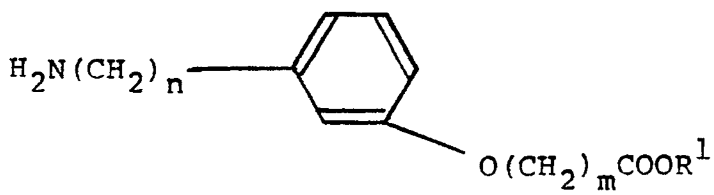

(3)

(wherein $R^1$ is $C_{1-4}$ alkyl) with a reactive derivative of a sulphonic acid $R^2SO_3H$;
(c) in the preparation of a compound in which $R^1$ is a hydrogen atom, hydrolysing a corresponding ester;
(d) in the preparation of a compound in which $R^1$ is a $C_{1-4}$ alkyl group, esterifying the corresponding carboxylic acid;
(e) in the preparation of a compound in which $-(CH_2)_n-$ is $-(CH_2)_3-$ or $-(CH_2)_4-$ and $R^2$ is other than thienyl, phenyl substituted by nitro, or $\beta$-styryl, reducing a compound of formula (4)

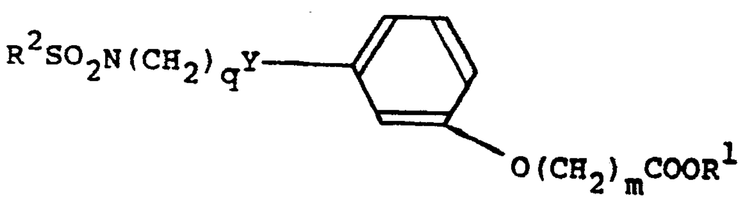

(4)

(wherein q is an integer 1 or 2, Y is $-CH=CH-$ or $-C\equiv C-$ and $R^2$ is as just defined):

(f) in the preparation of a compound in which $R^2$ is phenyl substituted by a hydroxyl group, demethylating the corresponding compound in which $R^2$ is phenyl substituted by a methoxy group; or

(g) in the preparation of a salt, treating a compound in which $R^1$ is a hydrogen atom with a base or treating a compound in which $R^2$ is phenyl substituted by amino with an acid or converting one salt into another.

**Claims for the following Contracting States: AT, GR, ES**

**1.** A process for the preparation of a compound of the general formula (1)

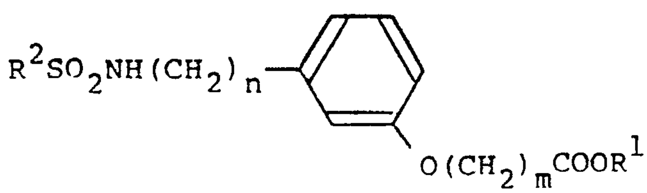

(1)

wherein

$R^1$ is a hydrogen atom or a $C_{1-4}$ alkyl group;

$R^2$ is a naphthyl, thienyl or $\beta$-styryl group

or a group $AR(CH_2)p$- where p is zero or an integer from 1 to 3 and Ar is phenyl or phenyl substituted by one or more halogen atoms or $C_{1-4}$ alkyl, $C_{1-4}$ alkoxy, hydroxyl, trifluoromethyl, nitro or amino groups;

m is an integer 1 or 2;

n is an integer from 1 to 4;

and the physiologically acceptable salts thereof,

which comprises:

(a) alkylation of a compound of formula (2)

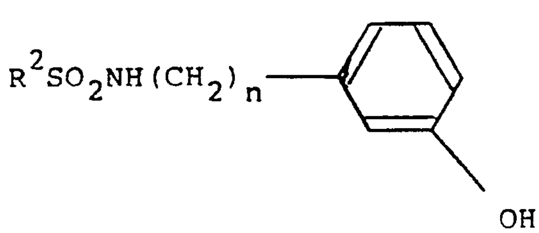

(2)

using an alkylating agent $L(CH_2)_mCOOR^1$ (where L is a leaving group);

(b) in the preparation of a compound in which $R^1$ is a $C_{1-4}$ alkyl group, sulphonylating a compound of formula (3)

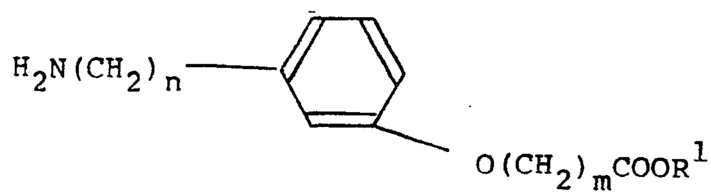

(3)

(wherein $R^1$ is $C_{1-4}$ alkyl) with a reactive derivative of a sulphonic acid $R^2SO_3H$;

(c) in the preparation of a compound in which $R^1$ is a hydrogen atom, hydrolysing a corresponding ester;

(d) in the preparation of a compound in which $R^1$ is a $C_{1-4}$ alkyl group, esterifying the corresponding carboxylic acid;

(e) in the preparation of a compound in which $-(CH_2)_n-$ is $-(CH_2)_3-$ or $-(CH_2)_4-$ and $R^2$ is other than thienyl, phenyl substituted by nitro, or $\beta$-styryl, reducing a compound of formula (4)

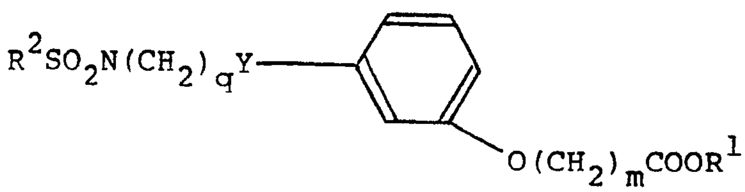

(4)

(wherein q is an integer 1 or 2, Y is -CH=CH- or -C≡C- and $R^2$ is as just defined):

(f) in the preparation of a compound in which $R^2$ is phenyl substituted by a hydroxyl group, demethylating the corresponding compound in which $R^2$ is phenyl substituted by a methoxy group; or

(g) in the preparation of a salt, treating a compound in which $R^1$ is a hydrogen atom with a base or treating a compound in which $R^2$ is phenyl substituted by amino with an acid or converting one salt into another.

**2.** A process according to claim 1 in which $R^1$ in the product is a hydrogen atom or a methyl group.

**3.** A process according to claim 1 or claim 2 in which m is in the product.

**4.** A process according to any preceding claim in which n is 2 or 3 in the product.

**5.** A process according to any preceding claim in which $R^2$ in the product is a naphthyl, phenyl or substituted phenyl group.

**6.** A process according to any of claims 1 to 4 in which $R^2$ in the product is a 2-naphthyl or phenyl group.

**7.** A process according to claim 1, in which the compound produced is [3-[3-[(phenylsulphonyl)amino]-propyl]phenoxy]acetic acid or a physiologically acceptable salt thereof.

**8.** A process according to claim 1, in which the compound produced is [3-[3-[(2-naphthalenylsulphonyl)-amino]propyl]phenoxy]acetic acid or a physiologically acceptable salt thereof.

**9.** A process for the preparation of a pharmaceutical composition which comprises formulating a compound of formula (1) as defined in claim 1 with one or more pharmaceutical carriers.

**10.** The use of a compound according to claim 1 for the manufacture of a pharmaceutical composition for the treatment of asthma and occlusive vascular diseases.

**Revendications**

**Revendications pour les Etats contractants suivants : BE, CH, DE, FR, GB, IT, LI, LU, NL, SE.**

**1.** Composés de formule développée (1)

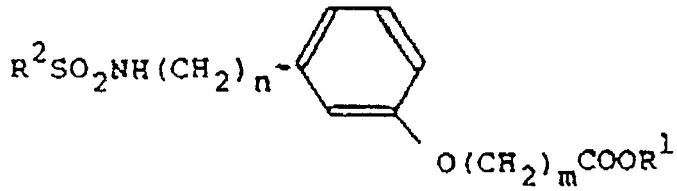

(1)

dans laquelle

$R^1$ est un atome d'hydrogène ou un groupe alcoyle ayant de 1 à 4 atomes de carbone ;

$R^2$ est un groupe naphtyle, thiényle ou $\beta$-styryle ou un groupe $Ar(CH_2)p$-, p étant zéro ou un nombre entier de 1 à 3 et Ar est phényle ou phényle substitué par un ou par plusieurs atomes d'halogène ou par des groupes alcoyle ayant de 1 à 4 atomes de carbone, alcoxy ayant de 1 à 4 atomes de carbone, hydroxyle, trifluorométhyle, nitro ou amino ;

m est un nombre entier égal à 1 ou 2 ;

n est un nombre entier allant de 1 à 4 ;

et leurs sels acceptables physiologiquement.

**2.** Composés suivant la revendication 1, dans lesquels $R^1$ est un atome d'hydrogène ou un groupe méthyle.

**3.** Composés suivant la revendication 1 ou la revendication 2, dans lesquels m est 1.

**4.** Composés suivant l'une quelconque des revendications précédentes, dans lesquels n est 2 ou 3.

**5.** Composés suivant l'une quelconque des revendications précédentes, dans lesquels $R^2$ est un groupe naphtyle, phényle ou phényle substitué.

**6.** Composés suivant l'une quelconque des revendications 1 à 4, dans lesquels $R^2$ est un groupe 2-naphtyle ou un groupe phényle.

**7.** Composé suivant la revendication 1, ce composé étant l'acide [3-[3-[(phénylsulphonyl)amino]propyl]-phénoxy]-acétique ou l'un de ses sels acceptables physiologiquement.

**8.** Composé suivant la revendication 1, ce composé étant l'acide [3-[3-[(2-naphthalénylsulphonyl)amino]-propyl]phénoxy]acétique ou l'un de ses sels acceptables physiologiquement.

**9.** Composition pharmaceutique comprenant un composé suivant l'une quelconque des revendications précédentes, associé à un ou à plusieurs Véhicules pharmaceutiques.

**10.** Procédé de préparation d'un composé suivant la revendication 1, qui consiste

(a) à alcoyler un composé de formule (2)

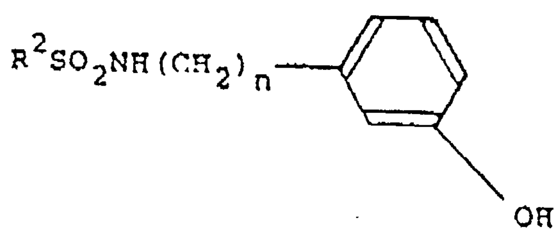

(2)

en utilisant un agent d'alcoylation $L(CH_2)_mCOOR^1$ (L étant un groupe labile) ;

(b) pour la préparation d'un composé dans lequel $R^1$ est un groupe alcoyle ayant de 1 à 4 atomes de carbone, à Sulfonyler un composé de formule (3)

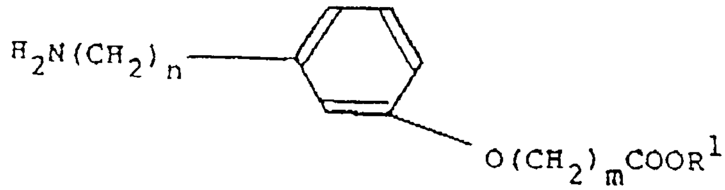

(3)

(dans laquelle $R^1$ est un alcoyle ayant de l à 4 atomes de carbone) par un dérivé réactif d'un acide sulfonique $R^2SO_3H$ ;

(c) pour la préparation d'un composé dans lequel $R^1$ est un atome d'hydrogène, à hydrolyser un ester correspondant;

(d) pour la préparation d'un composé dans lequel $R^1$ est un alcoyle ayant de l à 4 atomes de carbone, à estérifier l'acide carboxylique correspondant ;

(e) pour la préparation d'un composé dans lequel $-(CH_2)_n-$ est $-(CH_2)_3-$ ou $-(CH_2)_4-$ et $R^2$ est autre que thiényle, phényle substitué par nitro, ou $\beta$-styryle, à réduire un composé de formule (4)

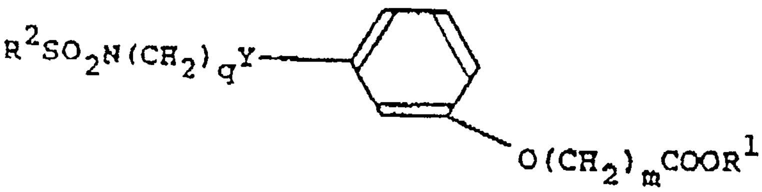

(4)

(dans laquelle q est un nombre entier égal & 1 ou 2, Y est -CH=CH- ou -C≡-C- et $R^2$ est tel qu'on vient de 1e définir) :

(f) pour la préparation d'un composé dans lequel $R^2$ est un phényle substitué par un groupe hydroxyle, à déméthyler le composé correspondant, dans lequel $R^2$ est phényle substitué par un groupe méthoxy ; ou

(g) pour la préparation d'un sel, à traiter un composé dans lequel $R^1$ est un atome d'hydrogène par une base ou à traiter un composé dans lequel $R^2$ est phényle substitué par un amino par un acide ou à transformer un sel en un autre.

**Revendications pour les Etats contractants suivants : AT, ES, GR**

1. Procédé de préparation d'un composé de formule développée (1)

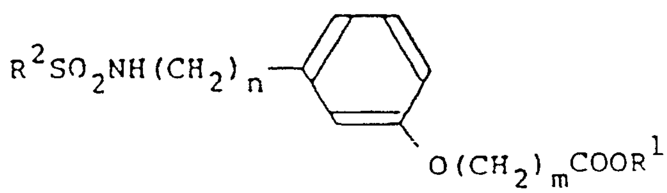

(1)

dans laquelle

$R^1$ est un atome d'hydrogène ou un groupe alcoyle ayant de 1 à 4 atomes de carbone ;

$R^2$ est un groupe naphtyle, thiényle ou $\beta$-styryle ou un groupe $Ar(CH_2)p-$, p étant zéro ou un nombre entier de 1 à 3 et Ar est phényle ou phényle substitué par un ou par plusieurs atomes d'halogène ou par des groupes alcoyle ayant de l à 4 atomes de carbone, alcoxy ayant de 1 à 4 atomes de carbone, hydroxyle, trifluorométhyle, nitro ou amino

m est un nombre entier égal & 1 ou 2 ;

n est un nombre entier allant de 1 à 4 ;

et de leurs sels acceptables physiologiquement, qui consiste :

(a) à alcoyler un composé de formule (2)

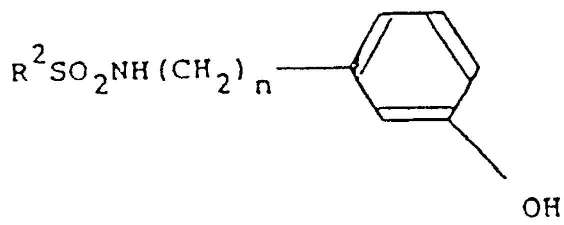

(2)

en utilisant un agent d'alcoylation $L(CH_2)_mCOOR^1$ (L étant un groupe labile) ;
(b) pour la préparation d'un composé dans lequel $R^1$ est un groupe alcoyle ayant de 1 à 4 atomes de carbone, à sulfonyler un composé de formule (3)

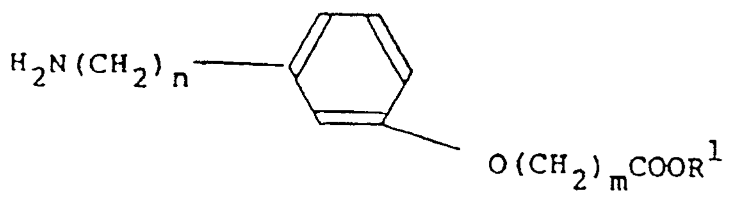

(3)

(dans laquelle $R^1$ est un alcoyle ayant de 1 à 4 atomes de carbone) par un dérivé réactif d'un acide sulfonique $R^2SO_3H$ :
(c) pour la préparation d'un composé dans lequel $R^1$ est un atome d'hydrogène, à hydrolyser un ester correspondant;
(d) pour la préparation d'un composé dans lequel $R^1$ est un alcoyle ayant de 1 à 4 atomes de carbone, à estérifier l'acide carboxylique correspondant ;
(e) pour la préparation d'un composé dans lequel - $(CH_2)_n$– est -$(CH_2)_3$- ou -$(CH_2)_4$- et $R^2$ est autre que thiényle, phényle substitué par nitro, ou $\beta$-styryle, à réduire un composé de formule (4)

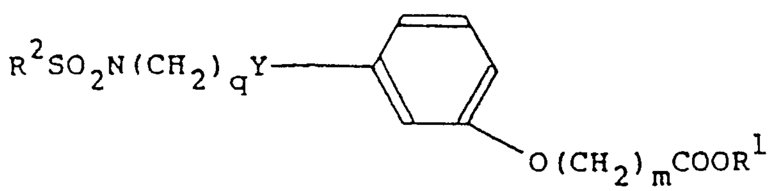

(4)

(dans laquelle q est un nombre entier égal à 1 ou 2, Y est - CH=CH- ou -C≡C- et $R^2$ est tel qu'on vient de le définir) :
(f) pour la préparation d'un composé dans lequel $R^2$ est un phényle substitué par un groupe hydroxyle, à déméthyler le composé correspondant, dans lequel $R^2$ est phényle substitué par un groupe méthoxy ; ou
(g) pour la préparation d'un sel, à traiter un composé dans lequel $R^1$ est un atome d'hydrogène par une base ou à traiter un composé, dans lequel $R^2$ est phényle substitué par un amino, par un acide ou à transformer un sel en un autre.

2. Procédé suivant la revendication 1, dans lequel $R^1$ dans le produit est un atome d'hydrogène ou un groupe méthyle.

3. Procédé suivant la revendication I ou la revendication 2, dans lequel m est égal à 1 dans le produit.

4. Procédé suivant l'une quelconque des revendications précédentes, dans lequel n est 2 ou 3 dans le produit.

5. Procédé suivant l'une quelconque des revendications précédentes, dans lequel $R^2$ dans le produit est un groupe naphtyle, phényle, ou phényle substitué.

6. Procédé suivant l'une quelconque des revendications 1 à 4, dans lequel $R^2$ dans le produit est un groupe 2-naphtyle ou un groupe phényle.

7. Procédé suivant la revendication 1, dans lequel le composé préparé est l'acide [3-[3-[(phénylsulfonyl)-amino]propyl]phénoxy]acétique ou l'un de ses sels acceptables physiologiquement.

8. Procédé suivant la revendication 1, dans lequel le composé préparé est l'acide [3-[3-[(2-naphténylsulfonyl)amino]propyl]phénoxy]acétique ou l'un de ses sels acceptables physiologiquement.

9. Procédé de préparation d'une composition pharmaceutique qui consiste à formuler un composé de formule (1) telle que définie à la revendication 1, avec un ou plusieurs véhicules pharmaceutiques.

10. Utilisation d'un composé suivant la revendication 1, pour la fabrication d'une composition pharmaceutique destinée au traitement de l'asthme et des troubles vasculaires occlusifs.

**Patentansprüche**
**Patentansprüche für folgende Vertragsstaaten : BE, CH, DE, FR, GB, IT, LI, LU, NL, SE**

1. Verbindungen der allgemeinen Formel (1)

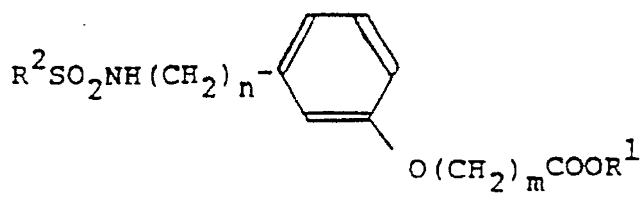

(1)

worin
$R^1$ ein Wasserstoffatom oder eine $C_{1-4}$-Alkylgruppe ist;
$R^2$ eine Naphthyl-, Thienyl- oder $\beta$-Styrylgruppe oder eine Gruppe $Ar(CH_2)p$- ist, worin p O oder eine Zahl von 1 bis 3 ist und Ar Phenyl oder Phenyl, substituiert durch ein oder mehrere Halogenatome, oder $C_{1-4}$-Alkyl-, $C_{1-4}$-Alkoxy-, Hydroxyl-, Trifluormethyl-, Nitro- oder Aminogruppen, ist;
m eine Zahl 1 oder 2 ist;
n eine Zahl von 1 bis 4 ist;
und die physiologisch annehmbaren Salze davon.

2. Verbindungen gemäß Anspruch 1, worin $R^1$ ein Wasserstoffatom oder eine Methylgruppe ist.

3. Verbindungen gemäß Anspruch 1 oder Anspruch 2, worin m = 1.

4. Verbindungen gemäß einem der vorhergehenden Ansprüche, worin n 2 oder 3 ist.

5. Verbindungen gemäß einem der vorhergehenden Ansprüche, worin $R^2$ eine Naphthyl-, Phenyl- oder substituierte Phenylgruppe ist.

6. Verbindungen gemäß einem der Ansprüche 1 bis 4, worin $R^2$ eine 2-Naphthyl- oder Phenylgruppe ist.

7. Verbindung gemäß Anspruch 1, wobei diese Verbindung [3-[3[(phenylsulfonyl)-amino]-propyl]-phenoxy]-essigsäure ist oder ein physiologisch annehmbares Salz davon.

8. Verbindung gemäß Anspruch 1, wobei diese Verbindung [3-[3-[(2-Naphthalinylsulfonyl)-amino]-propyl]-phenoxy]-essigsäure ist oder ein physiologisch annehmbares Salz davon.

9. Pharmazeutische Zusammensetzung, umfassend eine Verbindung gemäß einem der vorhergehenden Ansprüche, zusammen mit einem oder mehreren pharmazeutischen Trägern.

**10.** Verfahren zur Herstellung einer Verbindung gemäß Anspruch 1, welche umfaßt:
(a) Alkylierung einer Verbindung der Formel (2)

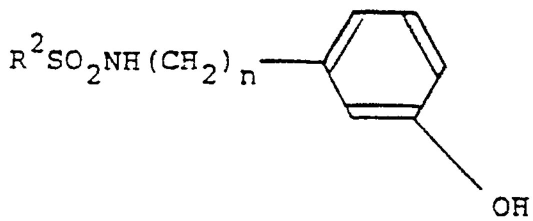

(2)

unter Verwendung eines Alkylierungsmittels $L(CH_2)_mCOOR^1$ (worin L eine austretende Gruppe ist);
(b) zur Herstellung einer Verbindung, worin $R^1$ eine $C_{1-4}$-Alkylgruppe ist, Sulfonieren einer Verbindung der Formel (3)

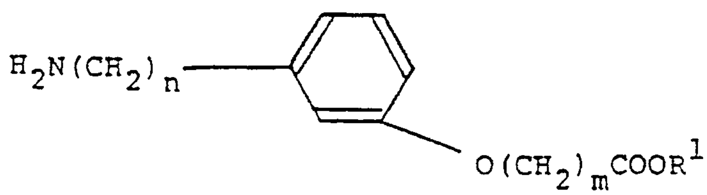

(3)

(worin $R^1$ eine $C_{1-4}$-Alkylgruppe ist) mit einem reaktiven Derivat einer Sulfonsäure $R^2SO_3H$;
(c) zur Herstellung einer Verbindung, worin $R^1$ ein Wasserstoffatom ist, Hydrolysieren eines entsprechenden Esters;
(d) zur Herstellung einer Verbindung, worin $R^1$ eine $C_{1-4}$-Alkylgruppe ist, Verestern der entsprechenden Carbonsäure;
(e) zur Herstellung einer Verbindung, worin $-(CH_2)n$ ist $-(CH_2)_3$-oder-$(CH_2)_4$- und $R^2$ anders als Thienyl, Phenyl substituiert durch Nitro, oder $\beta$-Styryl ist, Reduzieren einer Verbindung der Formel (4)

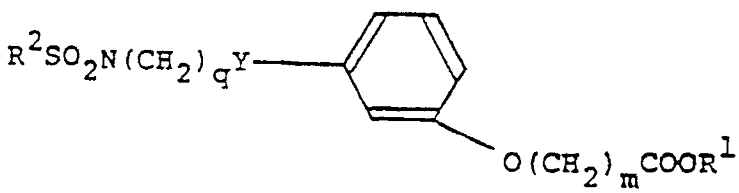

(4)

(worin q eine Zahl 1 oder 2 ist, Y -CH = CH- oder -C≡G- ist und $R^2$ wie eben definiert ist);
(f) zur Herstellung einer Verbindung worin $R^2$ Phenyl, substituiert durch eine Hydroxylgruppe, ist, Entmethylieren der entsprechenden Verbindung, worin $R^2$ Phenyl, substituiert durch eine Methoxygruppe, ist; oder
(g) zur Herstellung eines Salzes, Behandlung einer Verbindung, worin $R^1$ ein Wasserstoffatom ist, mit einer Base oder Behandlung einer Verbindung, worin $R^2$ Phenyl, substituiert durch Amino, ist mit einer Säure oder Umwandeln eines Salzes in ein anderes.

**Patentansprüche für folgende Vertragsstaaten : AT, ES, GR**

**1.** Verfahren zur Herstellung einer Verbindung der allgemeinen Formel (1)

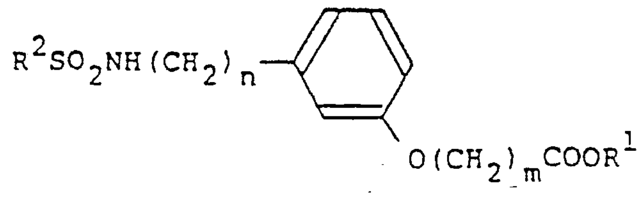

(1)

worin
$R^1$ ein Wasserstoffatom oder eine $C_{1-4}$-Alkylgruppe ist;
$R^2$ eine Naphthyl-, Thienyl- oder ß-Styrylgruppe oder eine Gruppe $Ar(CH_2)p$- ist, worin p O oder eine Zahl von 1 bis 3 ist und Ar Phenyl oder Phenyl, substituiert durch ein oder mehrere Halogenatome, oder $C_{1-4}$-Alkyl-, $C_{1-4}$-Alkoxy-, Hydroxyl-, Trifluormethyl-, Nitro- oder Aminogruppen, ist;
m eine Zahl 1 oder 2 ist;
n eine Zahl von 1 bis 4 ist;
und der physiologisch annehmbaren Salze davon, welches umfaßt:
(a) Alkylierung einer Verbindung der Formel (2)

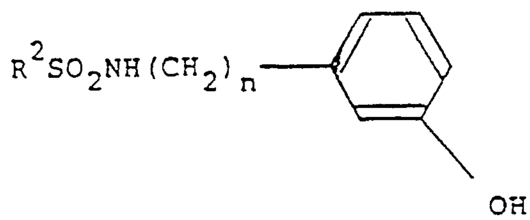

(2)

unter Verwendung eines Alkylierungsmittels $L(CH_2)_mCOOR^1$ (worin L eine austretende Gruppe ist);
(b) zur Herstellung einer Verbindung, worin $R^1$ eine $C_{1-4}$-Alkylgruppe ist, Sulfonieren einer Verbindung der Formel (3)

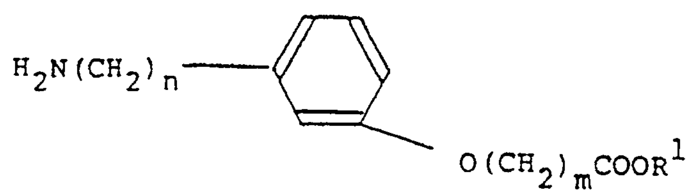

(3)

(worin $R^1$ $C_{1-4}$-Alkyl ist) mit einem reaktiven Derivat einer Sulfonsäure $R^2SO_3H$;
(c) zur Herstellung einer Verbindung, worin $R^1$ ein Wasserstoffatom ist, Hydrolysieren eines entsprechenden Esters;
(d) zur Herstellung einer Verbindung, worin $R^1$ eine $C_{1-4}$-Alkylgruppe ist, Verestern der entsprechenden Carbonsäure;
(e) zur Herstellung einer Verbindung, worin $-(CH_2)n$ ist $-(CH_2)_3$- oder $-(CH_2)_4$- und $R^2$ anders als Thienyl, Phenyl, substituiert durch Nitro, oder ß-Styryl ist, Reduzieren einer Verbindung der Formel (4)

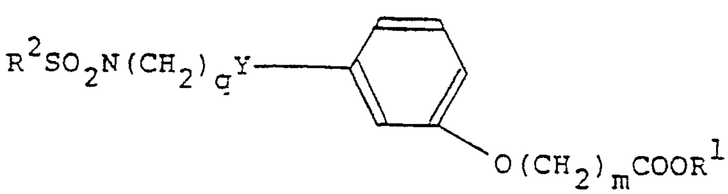

(4)

(worin q eine Zahl 1 oder 2 ist, Y -CH=CH- oder -C≡C- ist und $R^2$ wie eben definiert ist);
(f) zur Herstellung einer Verbindung, worin $R^2$ Phenyl, substituiert durch eine Hydroxylgruppe, ist,

Entmethylieren der entsprechenden Verbindung, worin $R^2$ Phenyl, substituiert durch eine Methoxygruppe, ist; oder
(g) zur Herstellung eines Salzes, Behandeln einer Verbindung, worin $R^1$ ein Wasserstoffatom ist, mit einer Base oder Behandlung einer Verbindung, worin $R^2$ Phenyl, substituiert durch Amino, ist mit einer Säure oder Umwandeln eines Salzes in ein anderes.

**2.** Verfahren gemäß Anspruch 1, worin $R^1$ in dem Produkt ein Wasserstoffatom oder eine Methylgruppe ist.

**3.** Verfahren gemäß Anspruch 1 oder Anspruch 2, worin $m = 1$ in dem Produkt ist.

**4.** Verfahren gemäß irgendeinem vorhergehenden Anspruch, worin n in dem Produkt 2 oder 3 ist.

**5.** Verfahren gemäß irgendeinem vorhergehenden Anspruch, worin $R^2$ in dem Produkt eine Naphthyl-, Phenyl- oder substituierte Phenylgruppe ist.

**6.** Verfahren gemäß irgendeinem der Ansprüche 1 bis 4, worin $R^2$ in dem Produkt eine 2-Naphthyl- oder Phenylgruppe ist.

**7.** Verfahren gemäß Anspruch 1, worin die hergestellte Verbindung [3-[3-[(Phenylsulfonyl)-amino]-propyl]-phenoxy]-essigsäure ist oder ein physiologisch annehmbares Salz davon.

**8.** Verfahren gemäß Anspruch 1, worin die hergestellte Verbindung [3-[3-[(2-Naphthalinylsulfonyl)-amino]-propyl]-phenoxy]-essigsäure ist oder ein physiologisch annehmbares Salz davon.

**9.** Verfahren zur Herstellung einer pharmazeutischen Zusammensetzung, welches umfaßt die Formulierung einer Verbindung der Formel (1), wie in Anspruch 1 definiert, mit einem oder mehreren pharmazeutischen Trägern.

**10.** Verwendung einer Verbindung gemäß Anspruch 1 zur Erzeugung einer pharmazeutischen Zusammensetzung zur Behandlung von Asthma und Gefäßverschlußkrankheiten.